# EUROPEAN PATENT SPECIFICATION

(11) **EP 1 571 911 B1**
(45) Date of publication and mention of the grant of the patent: **13.06.2012**
(21) Application number: 03811339.5
(22) Date of filing: 03.07.2003
(51) Int. Cl.: A01N 65/00

(54) **ARTHROPOD REPELLENT COMPRISING EXTRACTS AND/OR PARTS OF THE PLANT VITEX AGNUS-CASTUS**
ARTHROPODENREPELLIERENDE MITTEL ENTHALTEND EXTRAKTE UND/ODER TEILEN AUS DER PFLANZE VITEX AGNUS-CASTUS
REPULSIF POUR ARTHROPODES COMPRENANT DES EXTRAITS ET/OU DES PARTIES DE LA PLANTE VITEX AGNUS-CASTUS

(30) Priority: 19.11.2002 DE 10254072
(43) Date of publication of application: 14.09.2005
(73) Proprietor: Alpha-Biocare GmbH, 40225 Düsseldorf (DE); Meda AB, 170 09 Solna (SE)
(72) Inventor: BRUINS, Hans-Kervin, NL-7039 AA Stokkum (NL); MEHLHORN, Heinz, 41468 Neuss (DE); SCHMAHL, Günter, 50735 Köln (DE); SCHMIDT, Jürgen, 40221 Düsseldorf (DE)
(74) Representative: Gille Hrabal
(86) International application number: PCT/EP2003/007097
(87) International publication number: WO 2004/045291

(56) References cited:
- WO-A-00/64265
- ELGENGAIHI, S. E. ET AL: "Chemical and biological studies on Vitex agnus-castus L. volatile oils." INDIAN PERFUMER (1992), 36(4), 293-6 , 1992, XP009019282
- KUSTRAK D ET AL: "THE COMPOSITION OF THE ESSENTIAL OIL OF VITEX AGNUS-CASTUS" PLANTA MEDICA, THIEME, STUTTGART, DE, vol. 58, no. 1, 1992, page A681 XP000913595 ISSN: 0032-0943
- ZOGHBI MARIAS DAS GRACAS B ET AL: "The essential oil of Vitex agnus-castus L. growing in the Amazon region" FLAVOUR AND FRAGRANCE JOURNAL, vol. 14, no. 4, July 1999 (1999-07), pages 211-213, XP009019290 ISSN: 0882-5734
- DATABASE WPI Week 200145 Derwent Publications Ltd., London, GB; AN 2001-420779 XP002220310 "Food compositions containing Vitex agnus-castus fruits and isoflavones, useful remedies for premenstrual syndrome and female po puberal acne" & JP 2001 103931 A (FANCL CORP), 17 April 2001 (2001-04-17)
- HOBBS C: "The chaste tree: Vitex agnus castus." PHARMACY IN HISTORY. UNITED STATES 1991, vol. 33, no. 1, 1991, pages 19-24, XP009019241 ISSN: 0031-7047

## Description

### Field of the Invention

The present invention includes portions or extracts of semen of the plant Vitex agnus-castus (monk pepper) and their use as repellent against bothersome, lymph- and/or blood-sucking, skin penetrating, respectively food-, storage materials - or plant -damaging arthropods (mites, ticks, insects). Thus, this repellent protects the health of humans, pet animals and livestock, plants and stored materials.

### BACKGROUND OF THE INVENTION

### State of the Art

A large diversity of arthropods attack the skin of humans and animals in order to suck blood or lymph or lick body fluids or to dwell Into the skin in order to feed upon tissues. Such nuisant arthropods are mites (e.g. biting and scabies mites), ticks (e.g. members of the genera Ixodes, Rhipicephalus and argasids), mosquitoes (e.g, genera Anopheles, Culex, Aedes, Culiseta), simuliids, gnats or ceratopogonids, flies (e.g. genera Lucilia, Sarcophaga, Musca, Stomoxys, Phlebotomus, Glossina), tabanids, fleas (e.g. cat fleas, jigger fleas), bugs, lice (e.g. head, body, crab).

The members of the different groups of pests often occur in large numbers in nature and some enter buildings inhabited by humans and animals. They molest their host by flight attacks (e.g. mosquitoes in humans, flies in horses, simullids in cattle) and many are able to transmit a broad diversity of disease agents. Arthropod-borne diseases can led to severe illness and death of humans (e.g. malaria, yellow fever, sleeping sickness, Chagas disease) and animals (e.g. borreliosis, babesiosis, theileriosis). Impact by arthropods and diseases they transmit can also cause high economic losses (M. Rommel et al. 2000, Veterinärmedizinische Parasitologie, Parey, Berlin; H. Mehlhorn (ed.) 2001, Encyclopedic references of parasitology. Vol, 1 and 2, Springer, New York, Heidelberg; J.M. Lachapelle, D. Tennstedt, L. Marot, 1997, Atlas of environmental dermatology, ULC Press, Louvain, Brussels).

Therefore, the search for compounds with a long-lasting, highly effective and safe protective activity is as old as human culture (H. Schlicher 1984, Aetherische Ole: Wirkungen und Nebenwirkungen. Dt. Apothekerzeitschrift 124: 1433-1442). Compounds protecting against the attacks of ectoparasitic arthropods are called repellents (O. Hansen and M. Londershausen 2001: ectoparasiticides. In: H. Mehlhorn (ed.) 2001: Encyclopedic references of parasitology, Springer, Heidelberg, New York). In traditional medicine of many peoples all over the world extracts of plants were used as repellents against attacking arthropods. Especially essential oils from peppermint, geranium, lavender, citronella, thyme, lemongrass, cedar, and extracts of pennyroyal, eucalyptus and catnip have repellent properties (D.R. Barnard 1999. Repellency of essential oils to mosquitoes - diptera, Culicidae. J. Med. Entomol 36: 625-629; G. Nentwig 2003. Repellents. Parasitology Research 87: 214-216; H. Schlicher 1984. Aetherische Ole: Wirkungen und Nebenwirkungen. Dt. Apothekerzeitschrift 124: 1433-1442; A.Tawatsin et al. 2001, Repellency of volatile oils from plants against mosquito vectors. J. Vector Ecol). For example patent DE 3901341 A claims a mixture of citronellol and eugenol as repellent against tissue moths and cabinet beetles. However, to the prior art, plant derived products do not appear to repel insects as effective as chemicals like DEET. The majority of products marketed by the industry to repel or kill arthropods are based on chemically synthesized compounds (K.K. Buchel 1970, Chemistry of plant protection against pesticides, In: R. Wegler, Chemie der Planzenschutz- und Schddlingsbekdmpfungs-mittel, Vol. 1, Springer, Berlin, Heidelberg. New York). The most important synthetic repellent is N,N-diethyl-m-toluamide (DEET), which is contained in many products. Other registered repellents are dimethyl phthalate, 2-ethyl-hexane-1,3-diol, isopulegol, 1-piperidine carboxylic acid and hydroxy-ethyl-isobutyl-piperidine-carboxylate (M.S. Tradin 1998. Mosquitoes and mosquito repellents: a clinician's guide. Ann. Int, Med. 128: 931-940).

A severe disadvantage of all until now described repellant extracts from plants is the fact that they are predominantly effective only against selected groups of arthropods or a very limited spectrum of such targets. In addition, they have another important drawback:: the duration of protection that they confer is rather short (American Pharmaceutical Association, Washington D.C., Handbook of nonprescription drugs, 10th ed.). Furthermore, essential oils often smell disagreable and may be refuted by humans and animals. Some animals, notably cats, get sick upon contact with certain essential oils (e.g. tea-tree and thymian oil) (D. Villar et al, 1 994, Toxicity of Melaleuca oil and related essential oils applied on dogs and cats. Vet. Hum. Toxicol. 36: 139-142). These animals are unable to metabolize such oils due to the lack of certain necessary enzymes. Furthermore, essential oils have high potencies to provoke skin irritations and allergies, so that many humans cannot use them (A. Wolf 1999, Essential oil poisoning. J. Toxicol. Clin. Toxicol. 37: 721-727; S. Baum 2002, Aromatherapie: Zwischen Esotherik und Arzneimittelrecht, Pharm. Ztg. 147: 1208-1212). Essential oils easily permeate into the skin and also can carry other ingredients with them causing adverse effects (A.C. Williams, B.W. Barry 1992. Skin absorption enhancers. Crit. Rev. Ther. Drug Carrier Sys. 9: 17-24). DEET has the disadvantages that it has an unpleasant odor, attacks plastic materials, may harm health and, it has a low efficiency against ticks and mites (H. Quiu et al. 1998. Pharmacokinetics, formulation, and safety of insect repellent N,N-diethyl-3-methylbenzamide (DEET): a review. J. Am. Mosq. Contr. Assoc. 14: 12-27; P.J. Robbins, M.G. Cherniak 1986, Review of biodistribution and toxicology of the insect repellent N,N-dlethyl-3-m-tolulamide (DEET). J, Toxicol. Environ. Hlth. 18: 503-525). The compound Bayrepel hydroxy-ethyl-isobutyl-piperldine-carboxylate has much better properties as DEET, however, its activity against ticks is poor and it protects against them just for about two hours. Bayrepel clearly does not protect securely during typical several hours lasting outdoor activities especially in regions where many Ehrlichia-, virus- or Borrelia-infected Ixodes ticks occur.

In addition, none of the known plant extracts, DEET, Bayrepel, dimethyl phthalate and other synthetic repellents have a sufficient effectivity against flies which molest e.g. horses and humans enormously in rural regions. Also, known compounds of prior art have only a very limited activity against skin-penetrating, lymph- or bloodsucking arthropods. No compound is available which protects at the same time for at least 6 to 8 hours against the attacks of ticks, mites, mosquitoes, flies, tabanids, fleas, bugs and lice, smells well, does not harm the health of humans, dogs, cats, horses and other house animals and is based on a bioproduct (A. Turberg, 2001. Ectoparasiticides and repellents. In: H. Mehlhorn (ed.) 2001. Encyclopedic references of parasitology. Vol. 1 and 2, Springer, Heidelberg, New York; http//www.holzer,li/repellentien.htm). In spite that there are some compounds or combinations of plant extracts which have a good activity on a limited range of insects, there is not yet available a product, which protects equally efficient against all attacks of ticks, mites and various insects. However, such a broad-spectrum of protection is needed for usual outdoor activities, since the wanderer cannot foresee, whether he and his dog will be target either of flies, mosquitoes, fleas, ticks, mites, tabanids, gnats or all of them. Especially in the case of ticks, mites and flies there is no product on the market, that provides secure protection against attacks.

JP-A-07-0170710 discloses a vermin repellent containing 2-(2-formyl-3-methyl-cyclopent-2-enyl)acetaldehyde as an active component. The compound can be produced by extracting the leaf of fresh HAMAGO (Vitex rotundifolia), which is different from Vitex agnus-castus, collected in Tanegashima island with chloroform, distilling out the solvent, distilling the obtained concentrate under reduced pressure to obtain a volatile fraction and finally subjecting the fraction to silica gel thin-layer chromatography. The agent is told to be effective against blood-sucking vermin such as Aedes, Culices, gnat and Stomoxys calcitrans. No activity is however reported against for example ticks or mites, and its activity against these is in fact low or even not there.

Clearly there is a need for a repellent that is long-lasting effective against, in particular, ticks and insects, and that is save and pleasant to use. Thus, there is a strong need of a broad-spectrum repellent offering at the same time a protection of humans and animals against the until now not limited attacks of ticks, mites and flies.

The task of the present invention is the presentation of a long-lasting (i.e. 8 hours) broad-spectrum-repellent - being active as well against ticks and mites as against an extremely wide range insects. This problem is solved by the present invention as defined in the claims and the following description.

### SUMMARY OF THE INVENTION

Surprisingly it was found that the plant Vitex agnus-castus, including extracts, parts and other active components thereof, offer a long-lasting protection as well against ticks and mites as against a large variety of insects. This is not the case when using the known natural products or synthesized compounds. A further advantage of the present invention is that extracts of Vitex agnus-castus do not contain noxious constituents, which might harm the health of humans or animals, Vitex agnus-castus is a well known in phytomedicine for other types of oral treatments of patients. It is used to treat female hormonal disorders, e.g. premenstrual syndrome, menopausal symptoms, acne, or to promote milk flow (D. Brown 1994, Herbal Research review: Vitex agnus-castus Clinical Monograph.

Furthermore, extracts of leaves of Vitex agnus-castus have shown insecticidal activity against cotton leafworm (*Spodoptera litteralis*) (Elgengahi, S. E. et al.: Indian Perfumer (1992), 36(4), 293-6). Quarterly Review of Natural Medicine; DJ. Schellenberg 2001; Treatment for the premenstrual syndrome with agnus-castus fruit-extract: prospective, randomized, placebo controlled study. BMJ 322: 134-137). The - constituents of Vitex agnus-castus - since applied as herbal medicine since ancient times - had been thoroughly tested on found to be safe regarding side effects (J. Schellenberg 2001, see above; W. Hager 1990, Hager's Handbook of the Pharmaceutical Praxis, 5th ed.). Skin irritation and allergies, which may be provoked by application of several other repellents, that contain essential oils, are unknown to develop upon lang-lasting use of Vitex agnus-castus. In contrast to other repellents the extracts of Vitex agnus-castus do not smell strong. Therefore, it is easy to produce convenient repellents for daily use offering a high satisfaction to of the user. Extracts of Vitex agnus-castus can be used without any side effects on the health of humans and animals offering a broad spectrum protection against molesting arthropods (e.g. flies) or vectors of agents of diseases (e.g. borreliosis, ehrlichiosis via ticks, malaria, dengue, yellow fever, West nile fever via mosquitoes). This protection can be provided to both, humans and animals.

-Moreover, extracts of Vites agnus-castus are not detrimental to plants or natural storage materials. Hence, extracts of Vitex agnus-castus can be safely applied to protect such materials against pest organisms.

It is also an advantage that the plant Vitex agnus-castus is found in the wild throughout large regions at the Mediterranean Sea and in many regions of West Asia. The plant is also grown on commercial plantations in many subtropical countries. Thus, the raw material of Vitex agnus-castus are easily available for commercial expoitation. Of notable importance is the fact, that many consumers prefer nature-derived products over of synthetical chemicals.

### PRODUCTION OF REPELLENTS ACCORDING TO INVENTION

To produce the claimed repellents, the semen are used. One easy possibility of production starts with the powdering of the semen In a grinder and to extract the compounds by adding the plant powder organic solutions (e.g. acetonitrile. In this respect it is for example referred to US 2003/0054058 A1. Finally, the obtained extracts become mixed with different additives such as suitable carriers, including water, alcohols such as ethanol, isopropanol or glycerol, polyethyleneglycol, and tensids, emulgators, perfumes, thickening agents, stabilisators, antioxidants, fixatives or preservatives in order to obtain the final product. Favourable formulations of repellents from Vitex agnus-castus contain the oily extract of the plant in an amount of 0.05-100% (volume/volume), preferably 0.5-33% (v/v) and most preferably percentages of 1-7% (v/v).

### EXAMPLES

In order to demonstrate the unique capability of the inventive repellent the following examples are provided.

Semen of Vitex agnus-castus were powdered, 15 g of this powder were soaked in 75 ml acetonitrile and incubated overnight. The extract was filtrated through a paper filter and the solvent was completely evaporized at room temperature. One ml of the oily extract was solubilized with 4 ml of a solution consisting of 50% water, 20% genapol, 20% ethanol and 10% polyethyleneglycol 300. This repellent formulation was used for the following tests.

### 1. Tick species (Ixodes ricinus, Rhipicephalus sanguineus)

A. Balb/c mice (in a cage) were slightly covered (by spraying) with the repellent. Beginning immediately after the spraying and then after 1, 2, 3, 4, 5, 6, 7, 8 hours ticks were placed onto the hair of the mice. It was seen that these ticks immediately left the mice and hided in a corner for more than 8 hours, while after 4 hours 8 of 10 ticks on untreated mice were firmly attached to the skin.
B. The same experiment was done with two cats and two dogs with the same results. In all cases the ticks fled from the treated animals.

### 2. Flies (Sarcophaga carnaria, Grey flesh fly)

Hands covered with latex gloves were sprayed with the repellent or not (control). Then the hands were put into a cage with 500 adult flies for 3 minutes. The number of approaches, the touch downs and the time of sitting was noted, It turned out that during the first 3 hours many flies approached, but did not sit down. During the next 5 hours only 4-7 specimens out of 30 approaching ones touched the skin, but left it within 1-2 seconds. In the case of untreated control hands the flies approached from the very beginning and sat there for often more than 10-15 seconds.

### 3. Stable flies (Stomoxys calcitrans)

An uncovered hand was sprayed with the repellent. Then it was introduced into a cage containing 50 hungry flies at intervals of 30 minutes. The second hand of the same person remained untreated and was used as control. The approaches within 5 minutes were counted. It was noted that the stable flies did not approach to the treated hand for 4 hours and that later (until 7 hours) only a quarter or even only a tenth of the flies approached, while in the case of the control hand biting occurred immediately after introducing the hand into the cage.

### 4. Cat flea (Ctenocephalides felis)

Balb/c mice in a cage were sprayed with the repellent or not (controls) and put into cages with 50 hungry fleas. At intervals of 30 minutes it was controlled whether the fleas infested on the skin of the treated mice. While in the case of the untreated controls the fleas attached immediately to the mice, fleas left for 6 hours urgently the hair of treated mice in case they came into contact with these hair. For example after 6 hours of exposition 4 out of 50 fleas were found on the ground of the cage while one was free inside the hair and only one started sucking.

### 5. Mosquitoes (A. maculipennis, A. aegyptiand C. pipiens)

One the hand of a volunteer was sprayed with the repellent, the other hand not (control). The hand were introduced for 3 minutes at intervals of 1 hour into a cage containing about 500 hungry females. Within 8 hours no mosquito was seen to touch down on a treated region, while at the untreated hand blood sucking started immediately.

### 6. Lice (adults and nymphs of Pediculus humanus corporis)

On a piece of white tissue a circle was drawn with the repellent. Then 50 lice were placed into the center of this circle. Lice approaching the border of the circle stopped and moved immediately backwards. In case they were placed onto the sprayed place the left it immediately.

### 7. Moths (of cloths)

When moths were placed onto tissue treated with the repellent they left it immediately within the 6 hours of observation, while they stayed on untreated tissues for long.

In addition, numerous other tests were conducted with varying compostions and test animals, with the examples provided beling representative samples of the testing program which was conducted.

### Examples of formulation

Since a repellent - besides its claimed repellent activity - should have skin - caring - properties and propagation of well smelling odour, which are depending on personal preferences, there exist many possibilities of formulations. Thus the following examples are only few of the possible combinations:
1. Repellent as spray: odour nature, i.e. cool and smelling like meadow and forest
   10 g powdered semen of Vitex agnus-castus are mixed with 100 ml of 70% ethanol for 24 hours. Then the extract is filtered and is mixed without previous vaporization with the following ingredients: 33 ml ethanolic extract of Vitex agnus-castus,
   20 ml glycerol,
   perfumes, citric acid,
   water ad 100 ml
2. Repellent as spray with a note of refreshing citrus:
   33 ml ethanolic extract of Vitex agnus-castus,
   4 ml genapol®X-080 (Hoechst AG),
   0.3 ml lemongrass oil,
   water ad 100 ml

### 3. Repellent as lotion

In order to obtain the repellent by steam distillation 100 g of minced leafs of Vitex agnus-castus were mixed with 300 ml water in a distillery bottle, which is heated up (in a paraffin bath) to 120°C until the water is evaporized. The steam is collected and cooled down. The oily phase of the distilled product is separated from the aqueous layer. The following compounds were added one after the other and heated to 70°C:
2 ml oily extract of Vitex agnus-castus,
7 ml petrolatum,
5 ml glycerol,
5 ml isopropylpalmitate,
3 ml cetylalcohol,
1 ml di-stearyl dimethyl ammonium chloride,
1 ml dimethicone,
0.6 ml phenoxyethanol,
water ad 100 ml

### 4. Repellent against moths of clothes

10 g semen of Vitex agnus-castus were included into a closed paper sacklet. This sacklet is enveloped in an aluminium foil in order to avoid loss of odour when being stored prior to use,

## Claims

1. Extracts of semen of the plant Vitex agnus-castus, obtainable by powdering semen of Vitex agnus-castus, soaking the powder in acetonitrile, filtrating and evaporizing the solvent, for use as a repellent against infestation of arthropods.

2. Extracts according to claim 1 for use as a repellent against infestation of arthropods in humans and/or pet or food animals.

3. Extracts according to claim 1 for protection of plants or stored materials including textiles, furs, food and agricultural products.

4. Extracts according to any of any of claims 1 to 4 for use against ticks or mites.

5. Extracts according to any of claims 1 to 5, wherein the repellent additionally comprises at least one dermatologically acceptable carrier.

6. Extracts according to any of claims 1 to 6 for use as a repellent which is in the form of a solution, spray, aerosol, lotion, gel, cream, powder, perfume, sun filter or deodorant.

7. Extracts according to any of claims 1 to 7 for application on skin, hair, and/or clothes.

## Patentansprüche

1. Extrakte aus Samen der Pflanze Vitex agnus-castus, erhältlich durch Pulverisieren der Samen von Vitex agnus-castus, Einweichen des Pulvers in Acetonitril, Filtrieren und Verdampfen des Lösungsmittels, zur Verwendung als Repellent gegen Befall durch Arthropoden.

2. Extrakte nach Anspruch 1 zur Verwendung als Repellent gegen Befall durch Arthropoden bei Menschen und/oder Haustieren oder Nutztieren.

3. Extrakte nach Anspruch 1 zum Schutz von Pflanzen oder gelagerten Materialien einschließlich Textilien, Pelzen, Lebensmitteln und landwirtschaftlichen Produkten.

4. Extrakte nach einem der Ansprüche 1 bis 4 zur Verwendung gegen Zecken oder Milben.

5. Extrakte nach einem der Ansprüche 1 bis 5, wobei das Repellent zusätzlich mindestens einen dermatologisch verträglichen Träger umfasst.

6. Extrakte nach einem der Ansprüche 1 bis 6 zur Verwendung als Repellent, welches in Form einer Lösung, eines Sprays, eines Aerosols, einer Lotion, eines Gels, einer Creme, eines Pulvers, eines Parfums, eines Sonnenschutzfilters oder eines Deodorants vorliegt.

7. Extrakte nach einem der Ansprüche 1 bis 7 zur Anwendung auf der Haut, auf Haar und/oder auf Kleidung.

## Revendications

1. Extraits de semence de la plante Vitex agnus-castus qui peuvent être obtenir par pulvérisation de semence de Vitex agnus-castus, détrempage de la poudre dans l'acétonitrile, filtrage et evaporisation du solvant pour l'utilisation en tant qu'un répulsif contre l'infestation d'arthropodes.

2. Extraits de semence selon la revendication 1 pour l'utilisation en tant qu'un répulsif contre l'infestation d'arthropodes dans des humains et/ou des animaux domestiques ou des animaux d'élevage.

3. Extraits selon la revendication 1 pour la protection des plants ou des matériels stockés, comprenant des textiles, des fourrures, des alimentations et des produits agricoles.

4. Extraits selon l'une des revendications 1 à 4 pour l'utilisation contre les tiques ou les acariens.

5. Extraits selon l'une des revendications 1 à 5 dans lesquels le répulsif comprend en outre au moins un porteur dermatologiquement acceptable.

6. Extraits selon l'une des revendications 1 à 6 pour l'utilisation en tant qu'un répulsif qui est en forme d'une solution, d'un spray, d'un aérosol, d'une lotion, d'un gel, d'une crème, d'un poudre, d'un parfum, d'un filtre de soleil ou d'un déodorant.

7. Extraits selon l'une des revendications 1 à 7 pour l'application sur la peau, les chevaux et/ou les vêtements.
